# EUROPÄISCHE PATENTANMELDUNG

(11) **EP 2 138 095 A1**
(43) Veröffentlichungstag der Anmeldung: **30.12.2009**
(21) Anmeldenummer: 08158987.1
(22) Anmeldetag: 25.06.2008
(51) Int. Cl.: A61B 5/06, A61B 6/12, A61B 19/00

(54) **Verfahren zur Ermittlung der Position eines medizinischen Instruments in einem Körper**

(71) Anmelder: BrainLAB AG, 85622 Feldkirchen (DE)
(72) Erfinder: Schmidt, Robert, 80805 München (DE); Urban, Alexander, 85570 Markt Schwaben (DE); Vollmer, Fritz, 80469 München (DE)
(74) Vertreter: Schwabe - Sandmair - Marx

(57) **Zusammenfassung**

Die vorliegende Erfindung betrifft ein Verfahren zur Ermittlung der Position des Kopfes eines medizinischen Instruments in einem Gefäßsystem, wobei das Instrument in das Gefäßsystem eines Körpers einführbar ist und einen Pfad zumindest teilweise innerhalb des Gefäßsystems beschreibt und die Position des Instrumentenkopfes aus Strukturdaten, Längendaten sowie der relativen Position zwischen einem Referenzpunkt und dem Gefäßsystem berechnet wird, wobei das Instrument an dem Referenzpunkt vorbeigelührt wird, die Strukturdaten die Struktur des Gefäßsystems repräsentieren und die Längendaten die Länge des Pfades zwischen dem Referenzpunkt und dem Instrumentenkopf repräsentieren.

## Beschreibung

Die Erfindung betrifft ein Verfahren zur Ermittlung der Position eines Kopfes eines medizinischen Instruments in einem Körper, insbesondere in einem Gefäßsystem, ein Programm zur Durchführung des Verfahrens, ein Speichermedium, auf dem das Programm gespeichert ist, eine Vorrichtung zur Ermittlung der Position des Kopfes eines medizinischen Instruments in einem Körper sowie ein Operationssystem mit einem medizinischen Instrument und einer derartigen Vorrichtung.

Bei einer Operation oder einer Untersuchung werden oftmals medizinische Instrumente verwendet, die in den zu operierenden Körper, insbesondere ein Gefäßsystem, eingeführt werden. Dabei ist es wichtig, die Position des Kopfes, also des eingeführten Endes, des medizinischen Instruments zu kennen.

In der bisherigen Praxis erfolgt die Positionsbestimmung durch Echtzeit-Bildgebung mittels Röntgenverfahren, wobei kontinuierlich Röntgenbilder angefertigt werden. Dieses Vorgehen hat jedoch eine Strahlenbelastung sowohl für den Patienten als auch für den Arzt zur Folge.

Ein weiterer Ansatz, der in der US-Patentanmeldwng 200410171934 A1 offenbart wird, ist die Verwendung von elektromagnetischer Strahlung, die am Kopf des Instruments erzeugt wird. Dazu werden mehrere Spulen eingesetzt, die erstens in den Kopf des Instruments integriert und zweitens durch das Instrument mit Energie versorgt werden müssen. Dies kann ein größeres Instrument erfordern.

Es ist daher die Aufgabe der vorliegenden Erfindung, ein Verfahren und eine Vorrichtung zur Ermittlung der Position des Kopfes eines medizinischen Instruments in einem Körper bereitzustellen, bei dem eine Strahlungsbelastung verringert werden kann und insbesondere die Baugröße des medizinischen Instruments nicht erhöht werden muss. Letzterer ermöglicht eine breite Einsatzfähigkeit des Instruments.

Diese Aufgabe wird gelöst durch das Verfahren, das Programm, das Speichermedium, die Vorrichtung und das Operationssystem, die in den unabhängigen Ansprüchen angegeben sind. Vortcilhafte Ausgestaltungsformen sind den abhängigen Patentansprüchen zu entnchmen.

Eine Ausgestaltungsform der Erfindung betrifft ein Verfahren zur Ermittlung der Position des Kopfes eines medizinischen Instruments in dem Gefäßsystem. Das medizinische Instrument ist in ein Gefäßsystem eines Körpers einführbar. Wenn das Instrument eingeführt wird, beschreibt es einen Pfad zumindest teilweise innerhalb des Gefäßsystems. Dabei dringt das Instrument an einer Eintrittsstelle in das Gefäßsystem ein und folgt innerhalb des Körpers dem Verlauf des Gefäßsystems, wobei der Verlauf des Gefäßsystems den Pfad bestimmt, den das Instrument innerhalb des Körpers nimmt. Die Position des Kopfes des Instruments, im Folgenden als Instrumentenkopf bezeichnet, befindet sich innerhalb des Gefäßsystems. Der Kopf ist an dem eingeführten Ende des Instruments angeordnet und nimmt beispielsweise bis zu 1, 10 oder 25 Prozent der Länge des Instruments ein. Besitzt das Instrument keinen definierten Kopf, wie zum Beispiel ein Schlauch oder eine Nadel, so wird das Eingeführte Ende als Instrumentenkopf bezeichnet. Das Instrument ist bevorzugt flexibel, beispielsweise ein Katheter oder ein Endoskop.

Die Position des Instrumentenkopfes wird berechnet aus Strukturdaten, die die Struktur des Gefäßsystems repräsentieren, und Längendaten, die die Länge des Pfades zwischen einem Referenzpunkt, an dem das Instrument vorbeigeführt wird, und dem Instrumentenkopf repräsentieren, sowie der relativen Position zwischen dem Referenzpunkt und dem Gefäßsystem. Diese relative Position kann z. B. durch eine Position in einem Bezugssystem beschrieben werden, in dem ein Modell des Gefäßsystems ruht. Der Referenzpunkt entspricht bevorzugt der Eintrittsstelle des Instruments in dem Körper, kann jedoch auch jeder andere Punkt innerhalb oder außerhalb des Körpers sein, der auf dem Pfad des Instruments liegt. Der Pfad spiegelt die Form des Instruments in seiner Ausdehnung in Längsrichtung wieder, beispielsweise die Biegung eines schlauchförmigen Instruments.

Die Längendaten beschreiben demnach die Länge des Teils des Instruments zwischen dem Instrumentenkopf und dem Referenzpunkt. Der Referenzpunkt liegt in vorteilhafter Weise innerhalb des Gefäßsystems oder an der Eintrittsstelle des Instruments in das Gefäßsystems. Die Strukturdaten beinhalten den dreidimensionalen Aufbau des Gefäßsystems und werden bevorzugt vor dem Einführen des Instruments in das Gefäßsystem ermittelt, beispielsweise als Angiogramm durch Röntgenbestrahlung, Kernspin- oder eine Computertomographie. Die relative Position zwischen dem Referenzpunkt und dem Gefäßsystem stellt eine dreidimensionale Beziehung her, indem sie die Lage des Referenzpunktes bezogen auf das Gefäßsystem wiedergibt. Sie ist entweder vorbekannt oder wird ermittelt beziehungsweise gemessen. Eine Möglichkeit besteht darin, den Referenzpunkt manuell in den Strukturdaten zu markieren, beispielsweise bereits vor, während oder nach dem Einführen des Instruments. Eine andere Möglichkeit ist die Messung der Position des Körpers (und damit des Gefäßsystems) und des Relerenzpunktes mithilfe von Markervorrichtungen, wie sie später beschrieben werden.

Sind die relative Position zwischen Referenzpunkt und Gefäßsystem, die Struktur des Gefäßsystems (z. B. als Modell) und die Länge des Pfades zwischen Referenzpunkt und Instrumentenkopf bekannt, so existiert eine abgeschlossene Menge von Positionen innerhalb des Gefäßsystems, an denen sich der Instrumentenkopf befinden kann. Die reale Position ist abhängig von dem Pfad, den das Instrument im Gefäßsystem beschreibt. Dieser Pfad wird üblicherweise vor dem Einführen des Instruments in den Körper geplant, so dass daraus folgt, welche Position aus der Menge der möglichen Positionen der realen Position des Instrumentenkopfs im Gefäßsystem entspricht. Die berechnete Position des Instrumentenkopfes kann vom Arzt insbesondere bei der Navigation des Instruments genutzt werden. Insbesondere kann der Arzt die berechnete Position während des Einführvorgangs nutzen, um zu überprüfen, ob der geplante Pfad mit dem realen (tatsächlichen) Pfad, der aktuell gegeben ist, übereinstimmt. Wie hierzu die berechnete Position genutzt werden kann wird weiter unten erläutert.

Bevorzugt gehen in die Berechnung der Position des Instrumentenkopfes zusätzlich Abzweigungsdaten ein, die repräsentieren, welchen Abzweigungen das Instrument im Gefäßsystem gefolgt ist. Die Abzweigungsdaten sind zum Beispiel in einer Tabelle abgelegt, die enthält, bei welcher Pfadlänge eine Abzweigung erfolgt ist und in welche Richtung. Eine zweite Möglichkeit besteht darin, die Abzweigungen in den Strukturdaten zu markieren. Werden die Abzweigungsdaten bei der Berechnung berücksichtigt, so lässt sich beispielsweise aus der berechneten Position des Instrumentenkopfs erkennen, ob das Instrument dem vorher geplanten Pfad folgt. Die Abzweigungsdaten lassen sich beispielsweise auf Videobildern ermitteln, die während des Einführens des Instruments im Bereich des Instrumentenkopfes innerhalb des Gefäßsystems aufgenommen werden. Mit dem Begriff Videobild sind im Rahmen dieses Dokuments sowohl Standbilder als auch Bewegtbilder gemeint. Alternativ lassen sich die Abzweigungsdaten aus Ultraschallbildern Röntgenbildern, Kernspin- und Computertomograplaie-Aufnalamen oder Ausgangssignalen eines Gyrosensors am Instrumentenkopf gewinnen.

In einer bevorzugten Ausführungsform der Erfindung werden die Längendaten aus einer Relativbewegung zwischen dem Instrument und dem Referenzpunkt berechnet. Der Begriff Relativbewegung bezeichnet dabei die Gesamtheit aus einer zurückgelegten Distanz und einer Bewegungsrichtung. Hat sich das Instrument um eine gewisse Weglänge gegenüber dem Referenzpunkt bewegt, so hat sich der Instrumentenkopf um eben diese Weglänge tiefer in das Gefäßsystem hinein oder aus dem Gefäßsystem heraus bewegt. Dabei muss die Messung der Relativbewegung nicht zwangsläufig direkt am Referenzpunkt selber erfolgen, sondern kann auch an anderer Stelle, also indirekt, erfolgen. Befindet sich der Referenzpunkt beispielsweise innerhalb des Gefäßsystems, so kann die Relativbewegung auch zwischen dem Instrument und beispielsweise dem Eintrittspunkt des Instruments in den Körper ermittelt und der Relativbewegung zwischen dem Instrument und dem Referenzpunkt gleichgesetzt werden. Denn es kann davon ausgegangen werden, dass die Relativbewegung zwischen dem Instrument und dem Eintrittspunkt gleich der Relativbewegung zwischen dem Instrument und jeder anderen Position entlang des Pfades, also auch dem Referenzpunkt, ist.

Bevorzugt wird die Relativbewegung durch eine optische Messung ermittelt, insbesondere aus einer Markierung am Instrument. Bei der Markierung kann es sich beispielsweise um einen Balkencode, einen Strichcode, einen Farbcode oder eine reflektierende Markierung handeln. In die Markierung kann ein absoluter Längenwert codiert sein. Bei der Markierung kann es sich auch um ein regelmäßiges Muster handeln, dessen Relativbewegung in Bezug auf den Referenzpunkt zur Länge des Pfades kumuliert wird. Weiterhin ist eine Kombination aus Bilderfassung und Bildverarbeitung möglich, wie es beispielsweise bei einer optischen Computermaus durchgeführt wird.

Alternativ oder zusätzlich zu einer optischen Messung kann die Relativbewegung auch durch eine mechanische Messung ermittelt werden, beispielsweise mittels eines Abnehmerrades, das auf dem Instrument abrollt.

Eine zweite Ausgestaltungsform der vorliegenden Erfindung betrifft ein Verfahren zur Ermittlung der Position eines Kopfes eines medizinischen Instruments, das in einen Körper einführbar ist und einen Pfad zumindest teilweise innerhalb des Körpers beschreibt, -. Im Vergleich zur ersten Ausgestaltungsform ist der Pfad des Instruments nicht darauf beschränkt, der Struktur eines Gefäßsystems zu folgen.

Die Position des Instrumentenkopfes wird berechnet aus Verlaufsdaten, die den Verlauf des Pfades repräsentieren, und Längendaten, die die Länge des Pfades zwischen einem Referenzpunkt, an dem das Instrument vorbeigeführt wird, und dem Instrumentenkopf repräsentieren, sowie der relativen Position zwischen dem Referenzpunkt und dem Körper. Dabei wird eine Relativbewegung zwischen dem Instrument und dem Referenzpunkt automatisch ermittelt und die Längendaten werden aus der Relativbewegung automatisch berechnet.

Diese Ausgestaltungsform der Erfindung eignet sich insbesondere für starre medizinische Instrumente wie beispielsweise Biopsienadeln oder Schrauben. Der Pfad eines starren medizinischen Instruments entspricht einer Geraden und ist somit bekannt.

Die im Folgenden beschriebenen Ausbildungen sind sowohl auf die erste als auch auf die zweite Ausführungsform anwendbar.

Die Position der Eintrittsstelle und damit eines möglichen Referenzpunktes bezogen auf den Körper bzw. das Gefäßsystem kann anhand einer Markervorrichtung, z. B eines Markersterns oder einzelner (ortsfest zueinander angeordneter) Markerelemente, bestimmt werden. Bei einem Markerstern handelt es sich um ein Objekt mit drei oder mehr räumlich angeordneten, fest miteinander verbundenen Kugeln. Die Positionen der Kugeln im Raum sind beispielsweise mittels einer 3D-Kamera erfassbar. Da die relativen Positionen der Kugeln zueinander bekannt sind, kann aus den Positionen der Kugeln sowohl die Position als auch die Orientierung des Markersterns und damit die Position eines mit dem Markerstern gekennzeichneten Objekts ermittelt werden. Ein solches Objekt ist beispielsweise eine Vorrichtung zur Messung der relativen Position zwischen dem Referenzpunkt und dem Gefäßsystem beziehungsweise dem Körper. Diese Vorrichtung wird bevorzugt an der Eintrittsstelle des Instruments auf den Körper aufgesetzt. Insbesondere ist die relative Position zwischen der Markervorrichtung und dem Referenzpunkt bekannt, so dass durch Detektion der Markervorrichtung die Position des Referenzpunktes insbesondere relativ zum Pfad bestimmt werden kann.

In einer .Ausgestaltung der Erfindung wird ein Signal erzeugt, wenn die berechnete Position des Instrumentenkopfes einer vorgegebenen Position entspricht. Bei der vorgegebenen Position handelt es sich beispielsweise um die gewünschte Endposition des Instrumentenkopfes oder eine (mögliche) Abzweigung im Gefäßsystem. Bei dem Signal handelt es sich beispielsweise um ein Hinweis- oder Warnsignal an den Arzt oder um ein Spülsignal, dass das Einleiten einer Spülflüssigkeit durch das Instrument auslöst, um die Aufnahme eines Videobildes im Bereich des Instrumentenkopfes zu ermöglichen.

In einer weiteren Ausprägung der vorliegenden Erfindung wird die tatsächliche (aktuelle) Position des Instrumentenkopfes ermittelt. Dies geschieht beispielsweise mittels bildgebender Verfahren wie Röntgen oder Computertomographie oder elektromagnetischer Positionsbestimmung mittels stromdurchflossencr Spulen, deren Feld detektiert wird. Die tatsächliche Position ist diejenige Position, die der lnstrumentenlcopf aktuell in der Realität einnimmt. Die tatsächliche Position wird beispielsweise dann ermittelt, wenn die berechnete Position einer kritischen Position entspricht, zum Beispiel der gewünschten Endposition des Instrumentenkopfes oder einer Abzweigung im Gefäßsystem. Da die tatsächliche Position während des Einführens des Instruments nicht oder nur selten und in größeren zeitlichen Abständen erfolgt als bei einer rein röntgenbasierten Positionsermittlung, ist selbst bei der Verwendung eines Röntgenverfahrens die Strahlenbelastung sowohl für den Patienten als auch für den Arzt erheblich reduziert.

In einer bevorzugten Ausführungsform der Erfindung werden der Referenzpunkt und/oder die Längendaten mit Hilfe der tatsächlichen Position des Instrumentenkopfes angepasst. Dies erfolgt beispielsweise dadurch, dass die tatsächliche Position des Instrumentenkopfes als Referenzpunkt und die (vom Instrumentenkopf zurückgelegte) Pfadlänge in den Längendaten auf Null gesetzt werden. Dadurch wird ein neuer Ausgangspunkt für das erfindungsgemäße Verfahren geschaffen, der zukünftigen Positionsberechnungen zugrunde gelegt wird.

Alternativ oder zusätzlich kann eine Modifikation der Längendaten erfolgen. Eine Möglichkeit besteht darin, die Längendaten durch theoretische Längendaten zu ersetzen. Die theoretischen Längendaten sind diejenigen Längendaten, die zu einer Übereinstimmung der berechneten Position und der tatsächlichen Position führen würden, wenn sie der Berechnung zugrunde gelegt würden. Diese theoretischen Längendaten werden dann bei einer zukünftigen Bewegung des Instruments als Basis verwendet, beispielsweise bei der Aktualisierung der Längendaten aufgrund einer Relativbewegung zwischen dem Referenzpunkt und dem Gefäßsystem beziehungsweise dem Körper. Eine zweite Möglichkeit besteht darin, einen Skalierungsfaktor zu berechnen, mit dem die Pfadlänge multipliziert wird. Der Skalierungsfaktor wird so bestimmt, dass die berechnete Position der tatsächlichen Position entspräche, wenn der Berechnung die mit dem Skalierungsfaktor multiplizierte Pfadlänge zugrunde gelegt würde. Bei zukünftigen Positionsberechnungen geht in die Längendaten die mit dem Skalierungsfaktor multiplizierte Pfadlänge ein.

Die Erfindung betrifft weiterhin ein Programm, dass, wenn es in einer Datenverarbeitungseinrichtung geladen wird oder auf einer Datenverarbeitungseinrichtung läuft, die Datenverarbeitungseinrichtung veranlasst, das vorstehend beschriebene Verfahren auszuführen. Die Erfindung betrifft außerdem ein Speichermedium, auf dem ein solches Programm gespeichert ist, oder eine Signalwelle, die Informationen trägt, die ein solches Programm darstellen.

Die Erfindung betrifft weiterhin eine Vorrichtung zur Ermittlung der Position des Kopfes eines medizinischen Instruments, aufweisend einen Computer, auf dem das vorstehend beschriebene Programm läuft oder geladen ist.

Die Erfindung betrifft weiterhin ein Operationssystem mit einem medizinischen Instrument und einer Vorrichtung wie vorstehend beschrieben. In einer Ausgestaltungsform der Erfindung weist das Operationssystem eine Kamera im Bereich des Instrumentenkopfes zur Erfassung eines Videobildes auf. Das Videobild zeigt eine Innenansicht des Körpers bzw. des Gefäßsystems und kann sowohl zur Ermittlung der tatsächlichen Position des Instrumentenkopfes als auch bei der Navigation des Instruments, beispielsweise an Abzweigungen des Gefäßsystems, verwendet werden. Zeigt das Videobild beispielsweise eine Abzweigung des Gefäßsystems, obwohl an der berechneten Position des Instrumentenkopfes keine Abzweigung vorhanden ist, entspricht der Pfad des Instruments nicht dem gewünschten Pfad. An der Pfadlänge und der Information, dass sich der Instrumentenkopf an einer Abzweigung befindet, lässt sich eine Menge an Pfaden ermitteln, die das Instrument im Gefäßsystem beschreibt. Aus dieser Menge kann der Arzt den tatsächlichen (aktuellen) Pfad des Instruments bestimmen, beispielsweise anhand der Größe und/oder Struktur der Gefäße vor und/oder hinter der Abzweigung.

Eine Abweichung des tatsächlichen Pfades vom gewünschten Pfad liegt auch dann vor, wenn das Videobild keine Abzweigung zeigt, obwohl sich die berechnete Position des Instrumentenkopfes an einer Abzweigung befindet.

In einer weiteren Ausführungsform weist das Operationssystem eine Einrichtung zur Bestimmung der tatsächlichen Position des Instrumentenkoples auf. Bei dieser Einrichtung handelt es sich beispielsweise um ein Röntgcngerät, einen Computertomographen oder ein elektromagnetisches Positionsermittlungssystem.

Bevorzugt weist das Operationssystem eine auf den Körper gerichtete Kamera zur Erfassung eines Bildes, aus dem die Längendatcn ermittelbar sind, auf. Bei der Kamera handelt es sich beispielsweise um eine Itifrarotkainera. Eine Intrarotkamera hat den Vorteil, dass ihre Empfindlichkeit im nichtsichtbaren Frequenzspektrum liegt und eine zusätzliche Ausleuchtung in diesem Frequenzbereich den Arzt und den Patienten nicht beeinträchtigt.

Aus dem Bild der Kamera sind die Längendaten beispielsweise dadurch ermittelbar, dass eine Markierung in Form eines Balkencodes, eines Strichcodes, eines Farbcodes oder einer reflektierenden Markierung auf dem Instrument detektiert wird, die insbesondere eine zurückgelegte Pfadlänge repräsentieren. Bei einer Markierung kann es sich auch um einen Markerstern handeln, wie er vorstehend bereits beschrieben wurde. Der Marker ist dann an einem Teil des Instruments angeordnet, der nicht in den Körper eingeführt wird. Die Kamera befindet sich demnach außerhalb des Körpers und erfasst die Markierung auf dem Instrument. Weiterhin kann ein Marker als Referenz zur Markierung der Eintrittsstelle vorhanden sein. Damit ist beispielsweise auch eine Torsion zwischen dem Instrument und der Eintrittsstelle detektierbar.

Die vorliegende Erfindung soll anhand eines Ausführungsbeispiels näher erläutert werden. Dabei zeigt
- Figur 1: ein erfindungsgemäßes Operationssystem,
- Figur 2: ein Endoskop in einem Gefäßsystem und
- Figur 3: eine Biopsienadel.

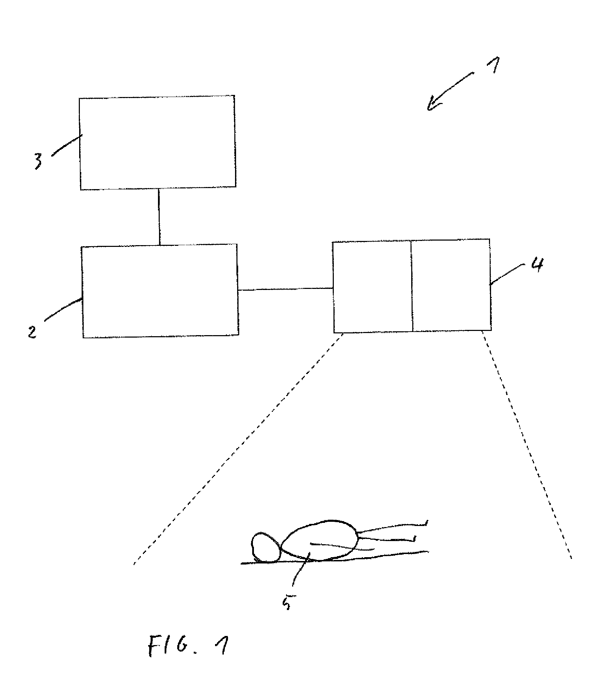

Figur 1 zeigt schematisch ein Operationssystem 1 mit einer Recheneinheit 2, einem Monitor 3 und einer 3D-Infrarotkamera 4. Im gestrichelt dargestellten Erfassungsbereich der Kamera 4 befindet sich ein zu untersuchender Patient 5.

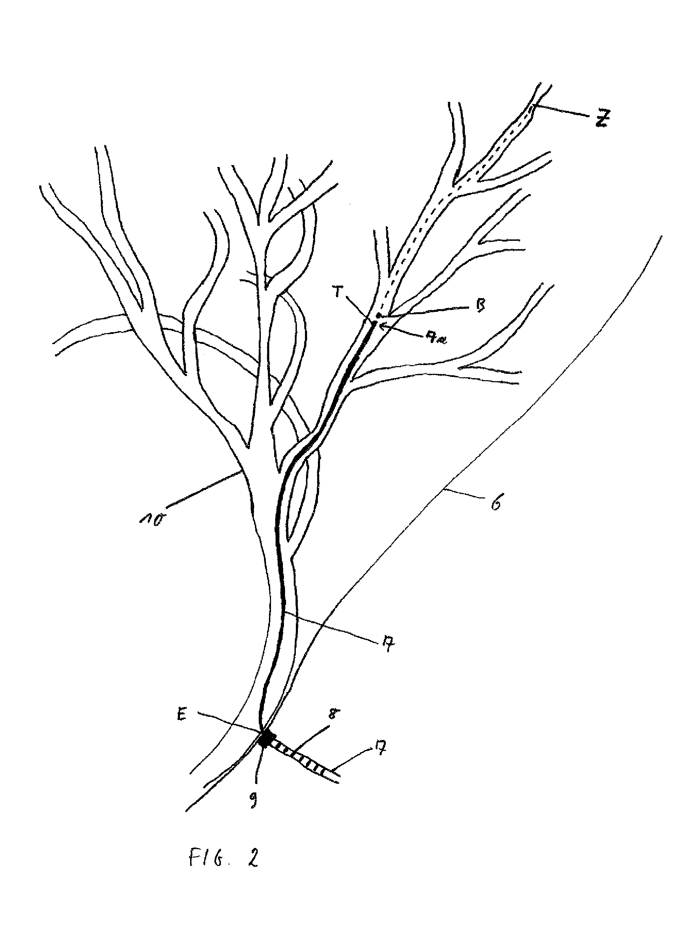

Dargestellt in Figur 2 ist ein Teil des unter der Haut 6 befindlichen Gefäßsystems 10 des Patienten 5. Das Gefäßsystem 10 soll an einer Position Z mittels eines Endoskops 7 untersucht werden. Das Endoskop 7 wird an einer Eintrittsstelle E in das unmittelbar unter der Haut 6 liegende Gefäßsystem 10 eingeführt. Die Struktur, also der dreidimensionale Aufbau, des Gefäßsystems 10 wurde vor dem Einführen des Endoskops 7 mittels eines Computertomographen ermittelt. Der geplante Pfad zwischen der Eintrittsstelle E und dem Zielpunkt Z ist gestrichelt dargestellt.

Auf dem Endoskop 7 befindet sich eine regelmäßige Markierung 8 in Form von äquidistanten Strichen, die von einer Messeinrichtung 9 an der Eintrittsstelle E erfasst werden. In der Meßeinrichtung 9 sind zwei Lichtquellen und zwei Fotodetektoren angeordnet, mit denen sich ermitteln lässt, in welche Richtung und um welche Weglänge das Endoskop 7 relativ zur Messeinrichtung 9 beziehungsweise der Eintrittsstelle E in das Gefäßsystem 10 eingeführt oder daraus herausgezogen wurde. Dazu ist die Messeinrichtung 9 mit der Recheneinheit 2 verbunden.

Die Position von Punkten, wie dem Referenzpunkt oder der Eintrittsstelle E, sowie die Position und/oder Orientierung von Objekten, wie dem Körper 5 oder der Messeinrichtung 9 können durch Markervorrichtungen oder einem Pointer gemessen oder direkt in die Recheneinheit 2 eingegeben werden. Ein Pointer ist eine Vorrichtung mit einer Markereinrichtung. Der Pointer wird an einen zu erfassenden Punkt gehalten und die Position des Punktes aus der Position der Markervorrichtung errechnet. Die Position der Markervorrichtung wird beispielsweise mithilfe der Kamera 4 ermittelt.

Der Eintrittspunkt E dient zunächst als Referenzpunkt für die Bestimmung der Position des Kopfes 7a des Endoskops 7. Der Recheneinheit 2 ist die Position der Eintrittsstelle E im Vergleich zum Patienten 5 und damit zum Gefäßsystem 10 bekannt, beispielsweise aufgrund von Messungen. Die Position und/oder Orientierung des Körpers 5 wird beispielsweise aus den Positionen charakteristischer Punkte des Körpers 5, sogenannter Landmarken, bestimmt. Alternativ befindet sich am Körper 5 ein Markerstern. Zu dem in Figur 2 dargestellten Zeitpunkt wurde der Instrumentenkopf 7a bis zur Position T in das Gefäßsystem 10 eingeführt. Auf dem Pfad zwischen dem Eintrittspunkt E und der Position T ist das Endoskop 7 den Abzweigungen links, rechts und links im Gefäßsystems 10 gefolgt.

Aus den Markierungen 8 auf dem Endoskop 7 haben die Messeinrichtung 9 und die Recheneinheit 2 Längendaten berechnet, die die Länge des Pfades zwischen dem Eintrittspunkt E und der Position T des Instrumentenkopfs 7a repräsentieren. Aus diesen Längendaten, zusammen mit der relativen Position zwischen dem Referenzpunkt E und dem Gefäßsystem 10 sowie Strukturdaten, die die Struktur des Gefäßsystems 10 repräsentieren, berechnet die Recheneinheit 2 die Position des Instrumentenkopfes 7a innerhalb des Gefäßsystems 10. Diese Position stimmt üblicherweise ausreichend genau mit der tatsächlichen Position des Instrumentenkopfes überein.

In der Figur 2 dargestellt ist ein Fall, bei dem die berechnete Position B nicht exakt mit der tatsächlichen Position T übereinstimmt, da der Pfad des Endoskops 7 im Gefäßsystem 10 nicht immer den kürzestmögliehen Verlauf nimmt. Bei einer Kontrollmessung mittels eines nicht dargestellten Röntgenapparatcs wird die tatsächliche Position T ermittelt und von der Recheneinheit 2 mit der berechnete Position B verglichen. Aufgrund der Abweichungen zwischen den beiden Positionen wird die tatsächliche Position T als neuer Referenzpunkt festgelegt. Bei zukünftigen Berechnungen der Position des Instrumentenkopfes 7a wird von diesem neuen Referenzpunkt T und der durch die Relativbewegung zwischen dem Endoskop 7 und dem neuen Referenzpunkt zurückgelegte Pfadlänge jenseits der Position R ausgegangen. Da die zurückgelegte Pfadlänge Relativbewegung nicht direkt an der Position T gemessen werden kann, erfolgt die Messung weiterhin am Eintrittspunkt E. Bei der weiteren Berechnung wird also davon ausgegangen, dass die Relativbewegung zwischen dem Endoskop 7 und dem Eintrittspunkt E der Relativbewegung zwischen dem Endoskop 7 und dem neuen Referenzpunkt T entspricht.

Ergibt die Positionsberechnung, dass sich der Instrumentenkopf 7a kurz vor der Zielposition Z befindet, so erzeugt die Recheneinheit 2 zum Beispiel ein Warnsignal für den Arzt und/oder ein Spülsignal, dass das Operationssystem 1 dazu veranlasst, eine Spülflüssigkeit in das Gefäßsystem 10 zu injizieren, um die Aufnahme eines Videobildes durch eine am Instrumentenkopf 7a befindliche Kamera zu ermöglichen.

Anstatt eines Endoskops 7 kann es sich auch um ein anderes flexibles medizinisches Instrument handeln, beispielsweise um einen Katheter. Im Gegensatz zu den Positionsermittlungsverfahren aus dem Stand der Technik weist das erfindungsgemäße Verfahren den Vorteil auf, dass die Position des medizinischen Instruments nicht kontinuierlich durch Röntgenaufnahmen bestimmt wird und somit sowohl der Arzt als auch der Patient einer Strahlenbelastung ausgesetzt werden, sondern eine Röntgenaufnahme nur optional zur Verifizierung der berechneten Position des Instrumentenkopfes verwendet wird.

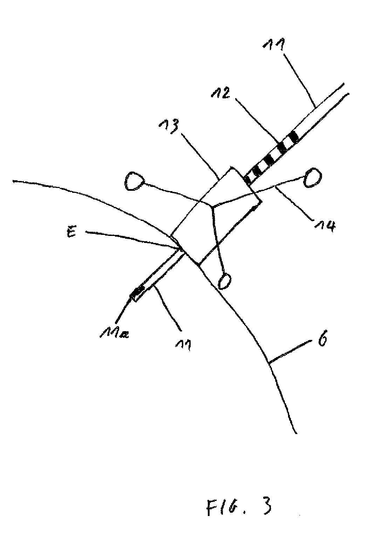

In dem Anwendungsbeispiel nach Figur 3 wurde eine Biopsienadel 11 mit einer Probenspitze 11a in den Körper des Patienten 5 eingeführt. Dazu wurde eine Einführeinrichtung 13, eine sogenannte Guiding Tube, an der Eintrittsstelle E auf die Haut 6 des Patienten 5 aufgesetzt. Die Biopsienadel 11 ist geradlinig durch die Einführeinrichtung 13 hindurchführbar. Optional fest mit der Einführeinrichtung 13 verbunden ist ein Markerstem 14, der drei räumlich angeordnete Kugeln aufweist. Die Kamera 4 erfasst die räumlichen Positionen der Kugeln. Aufgrund der bekannten Anordnung der Kugeln lassen sich aus dem 3D-Bild der Kamera 4 die Position und die Ausrichtung der Einführeinrichtung 13 bezogen auf den Körper des Patienten 5 eindeutig berechnen, wenn auch die Position und Orientierung des Körpers 5 bekannt sind. Diese werden beispielsweise mithilfe eines Markersterns oder der Position von Landmarken bestimmt.

Beim Einführen der starren Biopsienadel 11 in den Körper beschreibt die Prüfspitze 11a einen geradlinigen Pfad. Die Länge des Pfades, also die Eindringtiefe der Prüfspitze 11a bezogen auf die Haut 6, ermittelt die Einführeinrichtung 13 anhand eines Balkencodes 12 auf der Biopsienadel 11. Die Berechnung der Pfadlänge erfolgt gleich wie beim Anwendungsbeispiel nach Figur 2 mittels zweier Lichtquellen und zweier Photodetektoren.

In einer Alternative zu der dargestellten Ausführungsform wird die Relativbewegung zwischen dem Referenzpunkt E und der Biopsienadel 11 nicht in der Einführeinrichtung 13 bestimmt, sondern aus dem Bild der Kamera 4, die den Balkencode 12 auf der Biopsienadel 11 erfasst.

## Patentansprüche

1. Verfahren zur Ermittlung der Position des Kopfes (7a) eines medizinischen Instruments (7) in einem Gefäßsystem (10), wobei das Instrument (7) in das Gefäßsystem (10) eines Körpers (5) einführbar ist und einen Pfad zumindest teilweise innerhalb des Gefäßsystems (10) beschreibt und die Position des Instrumentenkopfes (7a) aus Strukturdaten, Längendaten sowie der relativen Position zwischen einem Referenzpunkt (E) und dem Gefaßsystem (7) berechnet wird, wobei das Instrument (10) an dem Referenzpunkt (E) vorbeigeführt wird, die Strukturdaten die Struktur des Gefäßsystem (10) repräsentieren und die Längendaten die Länge des Pfades zwischen dem Referenzpunkt (E) und dem Instrumentenkopf (7a) repräsentieren.

2. Verfahren nach Anspruch 1, **dadurch gekennzeichnet, dass** in die Berechnung der Position des Instrumentenkopfes (7a) zusätzlich Abzweigungsdaten eingehen, die repräsentieren, welchen Abzweigungen das Instrument (7) im Gefäßsystem (10) beim Einführen gefolgt ist.

3. Verfahren nach einem der Ansprüche 1 oder 2, **dadurch gekennzeichnet, dass** die Längendaten aus einer Relativbewegung zwischen dem Instrument (7) und dem Referenzpunkt (E) berechnet werden.

4. Verfahren nach Anspruch 3, **dadurch gekennzeichnet, dass** die Relativbewegung durch eine optische Messung ermittelt wird, insbesondere aus einer Markierung (8) am Instrument (7).

5. Veri-ahren zur Ermittlung der Position des Kopfes (11a) eines medizinischen Instruments (11) in einem Körper (5), wobei das Instrument (11) in den Körper (5) einführbar ist und einen Pfad zumindest teilweise innerhalb des Körpers (5) beschreibt und die Position des Instrumentenkopfes (11a) aus Verlaufsdaten, Längendaten sowie der relativen Position zwischen einem Referenzpunkt und dem Körper (5) berechnet wird, wobei das Instrument (11) an dem Referenzpunkt vorbeigeführt wird, eine Relativbewegung zwischen dem Instrument (11) und dem Referenzpunkt automatisch ermittelt wird, die Längendaten aus der Relativbewegung automatisch berechnet werden, die Verlaufsdaten den Verlauf des Pfades repräsentieren und die Längendaten die Länge des Pfades zwischen einem Referenzpunkt und dem Instrumentenkopf (11a) repräsentieren.

6. Verfahren nach einem der Ansprüche 1 bis 5, **dadurch gekennzeichnet, dass** ein Signal erzeugt wird, wenn die berechnete Position des Instrumentenkopfes (7a, 11a) einer vorgegebenen Position entspricht.

7. Verfahren nach einem der Ansprüche 1 bis 6, **dadurch gekennzeichnet, dass** die tatsächliche Position des Instrumentenkopfes (7a, 11a) ermittelt wird.

8. Verfahren nach Anspruch 7, **dadurch gekennzeichnet, dass** aus der tatsächlichen Position des Instrumentenkopfes (7a, 11a) der Referenzpunkt und/oder die Längendaten angcpasst werden.

9. Programm, das, wenn es in eine Datenverarbeitungseinrichtung geladen wird oder auf einer Datenverarbeitungseinrichtung läuft, die Datenverarbeitungseinrichtung veranlasst, das Verfahren nach einem der Ansprüche 1 bis 8 auszuführen.

10. Speichermedium, auf dem das Programm nach Anspruch 9 gespeichert ist, oder Signalwelle, die Informationen trägt, die das Programm nach Anspruch 9 darstellen.

11. Vorrichtung zur Ermittlung der Position des Kopfes (7a, 11a) eines medizinischen Instruments (7, 11), aufweisend einen Computer, auf dem das Programm nach Anspruch 9 läuft oder geladen ist.

12. Operationssystem (1) mit einem medizinischen Instrument (7, 11) und einer Vorrichtung nach Anspruch 11.

13. Operationssystem (1) nach Anspruch 12, **gekennzeichnet durch** eine Kamera im Bereich des Instrumentenkopfes (7a, 11a) zur Erfassung eines Videobildes.

14. Operationssystem (1) nach einem der Ansprüche 12 oder 13, **gekennzeichnet durch** eine Einrichtung zur Bestimmung der tatsächlichen Position des Instrumentenkopfes (7a, 11a).

15. Operationssystem (1) nach einem der Ansprüche 12 bis 15, **gekennzeichnet durch** eine auf den Körper (5) gerichtete Kamera (4) zur Erfassung eines Bildes, aus dem die Längendaten ermittelbar sind.
